# EUROPÄISCHE PATENTSCHRIFT

(11) **EP 1 198 494 B1**
(45) Veröffentlichungstag und Bekanntmachung des Hinweises auf die Patenterteilung: **19.01.2005**
(21) Anmeldenummer: 00949239.8
(22) Anmeldetag: 01.07.2000
(51) Int. Cl.: C08J 3/00, C08J 5/00

(54) **VERNETZERFREIE ZUBEREITUNGEN**
PREPARATIONS THAT ARE DEVOID OF CROSS-LINKING AGENTS
PREPARATIONS SANS RETICULANT

(30) Priorität: 12.07.1999 DE 19932076
(43) Veröffentlichungstag der Anmeldung: 24.04.2002
(73) Patentinhaber: Cognis Deutschland GmbH & Co. KG, 40589 Düsseldorf (DE)
(72) Erfinder: HEILEMANN, Andrea, D-89171 Illerkirchberg (DE); SANDER, Andreas, D-89257 Illertissen (DE); SCHÄFER, Gisbert, D-89165 Dietenheim (DE); HOLZER, Josef, D-89257 Illertissen (DE)
(86) Internationale Anmeldenummer: PCT/EP2000/006161
(87) Internationale Veröffentlichungsnummer: WO 2001/004187

(56) Entgegenhaltungen:
- EP-A- 0 538 904
- DATABASE WPI Week 198809 Derwent Publications Ltd., London, GB; AN 1988-061314 XP002153004 & JP 63 017901 A (HIGETA SHOYU KK), 25. Januar 1988 (1988-01-25) & PATENT ABSTRACTS OF JAPAN vol. 012, no. 223 (C-507), 24. Juni 1988 (1988-06-24) & JP 63 017901 A (HIGETA SHOYU KK), 25. Januar 1988 (1988-01-25) & CHEMICAL ABSTRACTS, vol. 108, no. 22, 30. Mai 1988 (1988-05-30) Columbus, Ohio, US; abstract no. 188789,
- CHEMICAL ABSTRACTS, vol. 111, no. 2, 10. Juli 1989 (1989-07-10) Columbus, Ohio, US; abstract no. 12542, "Chitosan sponges as surgical dressings." XP002153003 & JP 63 090507 A (UNITIKA LTD) 21. April 1988 (1988-04-21)
- PATENT ABSTRACTS OF JAPAN vol. 013, no. 260 (C-607), 15. Juni 1989 (1989-06-15) & JP 01 062302 A (NIPPON SUISAN KAISHA LTD), 8. März 1989 (1989-03-08) & CHEMICAL ABSTRACTS, vol. 111, no. 9, 28. August 1989 (1989-08-28) Columbus, Ohio, US; abstract no. 78544, "mANUFACTURE OF WATER SOLUBLE CHITOSAN SALTS" & DATABASE WPI Week 198916 Derwent Publications Ltd., London, GB; AN 1989-117701 & JP 01 062302 A (NIPPON SUISAN KAISHA LTD), 8. März 1989 (1989-03-08)
- PATENT ABSTRACTS OF JAPAN vol. 005, no. 180 (C-079), 19. November 1981 (1981-11-19) & JP 56 104902 A (KIMITSU KAGAKU KENKYUSHO:KK), 21. August 1981 (1981-08-21)

## Beschreibung

### Gebiet der Erfindung

Die Erfindung befindet sich auf dem Gebiet der Biopolymere und betrifft vemetzerfreie Zubereitungen, die man durch Fällung und anschließender Entwässerung von Biopolymeren erhält sowie ein Verfahren zu ihrer Herstellung.

### Stand der Technik

Aus der japanischen Patentanmeldung **JP-A2 Hei 6/048 917** (Nagawa) sind Schönheitspackungen mit Chitosan als aktiver Komponente sowie organischen Säuren und Kollagen als weitere Bestandteile bekannt. Gegenstand der japanischen Patentanmeldung **JP-A2 Hei 4/275 207** (Nitta Gelatin) sind feuchtigkeitsbindende Zusätze zu hautkosmetischen Mitteln, bei denen es sich um pulverförmige Mischungen von Chitosan und Kollagen handelt. Die europäische Patentanmeldung **EP A2 627 225** (Hüls) beschreibt Superabsorbentien aus mit Säure umgesetzten Chitosanen, die in Form eines Pulvers vorliegen.

Aus der deutschen Patentanmeldung **DE-A1 196 43 066** (Henkel) sind kollagenfreie kosmetische Zubereitungen bekannt, die durch Vernetzung von kationischen Biopolymeren mit Diisocyanaten und/oder Dialdehyden hergestellt werden. Das US-Patent **US 5,322,935** (AlliedSignal Inc.) beschreibt hochporöse, vemetzte Körper von Stickstoff enthaltende Polymere sowie ein Verfahren zu ihrer Herstellung. Bei diesem Verfahren wird ein Stickstoff enthaltendes Polymer zunächst in Wasser oder einer wässerigen Säure gelöst, dann mittels einer anionischen Salzlösung ionisch vernetzt, um abschließend mittels Vemetzungsmitteln kovalent vemetzt zu werden. Als Vemetzungsmittel werden hier beispielsweise Dialdehyde und aromatische sowie aliphatische Diisocyanate genannt. Die internationale Anmeldung **WO 96/20015** (Kimberly-Clark) beschreibt wasserquellbare, wasserunlösliche Chitosansalze mit einer definierten Absorptionskapazität bei externer Druckbelastung, die durch Vemetzung hergestellt werden können. In der japanischen Patentanmeldung **JP-A2 03165775** (Katakura Chikkarin Co.) wird die Herstellung von N-Succinyl-Chitosanen in Form eines multiporösen Schwammes oder Films durch Vemetzung mit Hexamethylendiisocyanat beschrieben. Diese Schwämme oder Filme eignen sich als prostethisches Material für Wundauflagen, künstliche Blutgefäße oder bfutstillende Auflagen. Das europäische Patent **EP-B1 663 212** (Hydromer Inc.) beschreibt Gele, die durch Vemetzung von Chitosan mit Polyvinylpyrrolidon erhalten werden.

Allen Produkten des Standes der Technik ist gemeinsam, daß der Verbund der Biopolymere durch chemische Vemetzung von reaktiven Zentren der Biopolymere erreicht wird. In der Regel werden zu diesem Zweck bifunktionelle Reagentien, wie beispielsweise Dialdehyde oder Diisocyanate eingesetzt. Da die vollständige Umsetzung dieser chemischen Vemetzer in der Regel nicht vorausgesetzt werden kann, verbleiben unter Umständen Reste dieser Vemetzer im Produkt. Dies kann, insbesondere bei Zubereitungen, die längere Zeit auf der Haut verbleiben, wie kosmetische Mittel oder Heilmittel, insbesondere Masken, oder Wundauflagen, Irritationen oder Allergien verursachen. Dies ist insbesondere bei Wundauflagen, die auf bereits gereizte oder geschädigte Haut aufgelegt werden, von großem Nachteil. Darüber hinaus ist durch den Zusatz dieser chemischen Vemetzer die biologische Abbaubarkeit beeinträchtigt.

Weiterhin können mit den üblichen chemischen Vemetzem vemetzte Produkte nicht als Nahrungsoder Nahrungsergänzungsmittel oder als Drug Carrier für orale Applikationen verwendet werden.

Die Aufgabe der vorliegenden Erfindung hat somit darin bestanden, vemetzerfreie Zubereitungen zur Verfügung zu stellen, die vergleichbare Eigenschaften, wie bekannte, unter Einsatz von Vernetzem hergestellte Zubereitungen, haben. Insbesondere soll eine dreidimensionale Struktur, in Form eines Blocks, eines Vlieses oder einer Maske, herstellbar sein. Besondere Beachtung finden hierbei Eigenschaften wie mechanische Stabilität in trockenem wie im nassem Zustand, Quellbarkeit als auch Kompatibilität mit weiteren möglichen Inhaltsstoffen. Des weiteren sollte die Herstellung einfach und je nach den gewünschten Eigenschaften des Endproduktes variabel sein. Wünschenswert ist weiterhin eine biologische Abbaubarkeit der Produkte.

### Beschreibung der Erfindung

Gegenstand der Erfindung sind vemetzerfreie Zubereitungen, dadurch erhältlich, daß man wässerige Lösungen und/oder homogenisierte Suspensionen von Biopolymeren mit Fällungsmitteln versetzt und anschließend entwässert.

Überraschenderweise wurde gefunden, daß die so erhältlichen Zubereitungen gegenüber bekannten, vemetzerhaltigen Zubereitungen vergleichbare Eigenschaften aufweisen. Insbesondere sind mit den erfindungsgemäßen Zubereitungen dreidimensionale Strukturen, wie Blöcke, Vliese oder Masken, herstellbar, die hinsichtlich ihrer mechanischen Stabilität, Elastizität, ihrer Quellbarkeit, Wasseraufnahmevermögen sowie ihrer Kompatibilität mit weiteren Inhaltsstoffen mit den Produkten des Stand der Technik vergleichbar sind. Die erfindungsgemäßen Zubereitungen weisen zudem eine hohe Hautverträglichkeit auf und sind biologisch abbaubar. Darüber hinaus sind sie technisch einfach herstellbar.

Die mechanische Stabilität der erfindungsgemäßen Zubereitungen, gemessen als Zugfestigkeit beim Bruch nach DIN 53 571, Probekörper B, liegt im Bereich von 10 und 1000 mN/mm², bevorzugt im Bereich von 50 bis 200 im trockenen Zustand und zwischen 10 und 500, bevorzugt zwischen 30 und 100 mN/mm² im nassen Zustand. Die Elastizität bestimmt als Dehnung beim Bruch (Methode nach DIN 53 571, Probekörper B) in % liegt zwischen 1 und 50, insbesondere zwischen 5 und 20 % im trockenen Zustand.
Die erfindungsgemäßen Zubereitungen haben eine Wasseraufnahmefähigkeit von mindestens 5 g Wasser / g Produkt, insbesondere von mindestens 15 g Wasser / g Produkt. Zur Bestimmung der Wasseraufnahme wird das Material mit entionisiertem Wasser befeuchtet und ausgewogen.

Ein weiterer Gegenstand der vorliegenden Erfindung ist ein Verfahren zur Herstellung vemetzerfreier Zubereitungen, bei dem man wässerige Lösungen und/oder homogenisierte Suspensionen von Biopolymeren mit Fällungsmitteln versetzt und anschließend entwässert.

Im Gegensatz zur chemischen Vernetzung beruht das vorliegende Verfahren auf der Erkenntnis, daß es durch die Zugabe der Fällungsmittel zu einer Verschiebung des pH-Wertes kommt, welche eine teilweise oder vollständige Ausfällung und gleichzeitig eine physikalische Vernetzung des Biopolymers zur Folge hat. Im Gegensatz zur chemischen Vernetzung kommt die Vernetzung der Fasern hierbei nicht durch kovalente Bindungen zustande, sondern vermutlich aufgrund von lonenpaarbildung, elektrostatischer Anziehung sowie mechanischer Verzwirbelung der Fasern.

Als vemetzerfrei im Sinne der vorliegenden Anmeldung ist somit zu verstehen, daß die mechanische Stabilität der Zubereitung vorrangig durch physikalische Vemetzung zustande kommt, insbesondere daß keine chemischen Vemetzer, wie bi- bzw. multifunktionale Reagenzien (beispielsweise Dialdehyde oder Diisocyanate) zur Vemetzung eingesetzt werden.

### Biopolymere

Unter dem Bergiff Biopolymere werden im Sinne der vorliegenden Anmeldung eine Gruppe von natürlich vorkommenden Makromolekülen zusammengefaßt, die wesentliche Bestandteile von lebenden Organismen sind und über Polykondensations-Reaktionen gebildet werden. Im Sinne der vorliegenden Erfindung können prinzipiell alle Biopolymere eingesetzt werden, die in wässeriger Lösung vorliegen bzw. die als wässerige Suspension hergestellt werden können und mit denen durch Zusatz eines Fällungsmittels eine physikalische Vemetzung stattfindet.

Besonders geeignet als Biopolymere im Sinne der vorliegenden Anmeldung sind Polysaccharide, wie beispielsweise Inulin, Mannane, Galactane, Xylane, Chitin, Chitosan, Cellulose, Pektine, Alginate, Carageene, Agar-Agar, Johanisbrotkemmehl sowie ihre Derivate, wie beispielsweise Carboxymethylcellulosen, anionisch oder nicht-ionisch derivatisierte Chitosane. Eine Vielzahl dieser Biopolmere tragen Carboxy- und/oder Sulfonylgruppen, was ihnen Polyelektrolytcharakter verleiht. Nach der Gesamtladung der Biopolymere kann man anionische, nicht-ionische und kationische Biopolymere unterscheiden.

### Wässerige Lösungen und/oder homogenisierte Suspensionen

Die Biopolymere werden als wässerige Lösungen und/oder homogenisierte Suspensionen eingesetzt. Die Suspensionen der Biopolymere enthalten in der Regel gelöste Anteile der Biopolymere. In der Regel werden kationische Biopolymere in wässerigen Mineralsäuren oder wässerigen organischen Carbonsäuren gelöst oder suspendiert. Als Mineralsäuren sind geeignet Salzsäure, Phosphorsäure, Salpetersäure sowie Schwefelsäure, als organische Carbonsäuren seien genannt: Ameisensäure, Milchsäure, Propionsäure, Maleinsäure, Brenztraubensäure, Glykolsäure, Bemsteinsäure, Essigsäure, Zitronensäure, Weinsäure sowie Adipinsäure. Besonders bevorzugt sind Salzsäure, Milchsäure sowie Glykolsäure. In der Regel werden anionische Biopolymere in wässrigen anorganischen Basen oder wässrigen organischen Stickstoffbasen gelöst oder suspendiert. Als anorganische Basen sind geeignet wässerige Lösungen von Carbonaten, Hydrogencarbonaten, Hydrogenphosphaten und Hydroxiden von Alkali- und Erdalkalimetallen, Ammoniak, sowie organische Stickstoffbasen, wie beispielsweise Triethylamin oder Triethanolamin.

Es werden die Mengen an Lösungsmittel eingesetzt, die zu einer teilweisen bis vollständigen Lösung der Biopolymere benötigt werden.

In der Regel wird eine 0,1 bis 15 Gew.-%ige wässerige Lösung bzw. Suspension mit gelösten Anteilen der Biopolymere eingesetzt, bevorzugt ist eine 0,5 bis 10 Gew.-%ige, insbesondere eine 1,0 bis 5,0 Gew.-% und insbesondere eine 1,5 bis 2,5 Gew.-%ige wässerige Lösung bzw. Suspension. Dabei hat es sich als vorteilhaft erwiesen, die Konzentration der wässerigen Lösung und/oder Suspension so einzustellen, daß die Lösung bzw. Suspension eine Viskosität im Bereich von 1000 bis 100 000 mPas (gemessen nach Brookfield, Temperatur von 20 °C) aufweist. Insbesondere eine Viskosität im Bereich von 10 000 bis 40 000 mPas, vorzugsweise im Bereich von 15 000 bis 35 000 mPas hat sich als vorteilhaft erwiesen. Handelt es sich um eine Suspension der Biopolymere, so kann es vorteilhaft sein, die Suspension zu homogenisieren, um die gewünschte Viskosität zu erhalten. Hierzu eigenen sich prinzipiell alle bekannten Methoden der Homogenisation, z.B. Kolloidmühlen oder Spalthomogenisatoren. Als besonders vorteilhaft hat sich der Einsatz einer Kolloidmühle zur Herstellung einer homogenisierten Suspension erwiesen. Die Homogenisation erfolgt in der Regel bei Temperaturen im Bereich von 0 bis 100 °C, insbesondere bei 30 bis 65 °C.

Die wässerige Lösung bzw. homogenisierte Suspension der Biopolymere hat je nach Art des Biopolymers und des verwendeten Lösungsmittels in der Regel einen pH-Wert im Bereich von 1 bis 12.

### Fällungsmittel

Als Fällungsmittel sind prinzipiell alle Stoffe geeignet, die den pH-Wert der wässerigen Lösung bzw. homogenisierten Suspension mit gelösten Anteilen der Biopolymere in den jeweils anderen pH-Bereich verschieben und dadurch eine zumindest teilweise Ausfällung und in der Folge daraus entstehenden physikalischen Vernetzung der Biopolymere bewirken. Zur Verschiebung des pH-Wertes vom alkalischen in den sauren Bereich eignen sich wässerige Lösungen von Mineralsäuren oder von organischen Carbonsäuren. Als Mineralsäuren sind beispielsweise geeignet Salzsäure, Phosphorsäure, Salpetersäure sowie Schwefelsäure, als organische Carbonsäuren seien genannt: Ameisensäure, Milchsäure, Propionsäure, Maleinsäure, Brenztraubensäure, Glykolsäure, Bernsteinsäure, Essigsäure, Zitronensäure, Weinsäure sowie Adipinsäure. Besonders bevorzugt sind Salzsäure, Milchsäure sowie Glykolsäure. Zur Verschiebung des pH-Wertes vom sauren in den alkalischen Bereich eignen wässerige anorganische Basen oder wässerige organische Stickstoffbasen. Als anorganische Basen sind geeignet wässerige Lösungen von Carbonaten, Hydrogencarbonaten, Hydrogenphosphaten und Hydroxiden von Alkali- und Erdalkalimetallen, Ammoniak, als organische Stickstoffbasen sind beispielsweise Triethylamin oder Triethanolamin geeignet. In einer bevorzugten Ausführungsform wird als anorganische Base Natriumhydrogencarbonat eingesetzt.

Die vorliegende Erfindung umfaßt die Erkenntnis, daß über das Verhältnis von Fällungsmittel zu vorliegendem Lösungsmittel die mechanischen Eigenschaften des Endproduktes beeinflußt werden können: wird eine vollständige Ausfällung des Biopolymers gewünscht, wird eine zur eingesetzten Säure bzw. Basemenge äquimolare Menge an Base bzw. Säure eingesetzt (in der Regel 0,8 - 1,2 mol Base : 1 mol Säure, insbesondere 0,9 - 1,1 mol Base : 1 mol Säure, und insbesondere 1 mol Base : 1 mol Säure und umgekehrt). Sind die Anforderungen an die mechanischen Eigenschaften des Endproduktes geringer, kann eine nur teilweise Ausfällung mit weniger als der äquimolaren Menge an Base bzw. Säure durchgeführt werden. Ist eine hohe Alkalität bzw. Acidität im Endprodukt erwünscht, kann ein molarer Überschuß an Base bzw. Säure eingesetzt werden.

Durch die Behandlung mit dem Fällungsmittel wird der pH-Wert der wässerigen Lösung bzw. homogenisierten Suspension der Biopolymere in der Regel je nach Lösungs- und Fällungssystem auf einen pH-Wert von 1 bis 14 eingestellt, insbesondere auf einen pH-Wert von 4,0 bis 8,5.

### Entwässerung

Die vorliegende Erfindung schließt die Erkenntnis ein, daß durch die Wahl der Methode der Entwässerung sowie durch die Parameter der jeweils gewählten Methode die mechanische Stabilität des Endproduktes beeinflußt werden kann.

Geeignete Methoden der Entwässerung sind beispielsweise die Lufttrocknung, die Vakuumtrocknung, insbesondere bei Temperaturen von 20 bis 100 °C oder über 100 °C sowie die Gefriertrocknung.

In einer bevorzugten Ausführungsform der vorliegenden Erfindung wird zur Entwässerung die Gefriertrocknung eingesetzt.

Als besonders geeignet hat es sich erwiesen, der Entwässerung einen Einfrierschritt voranzustellen. Dies ist insbesondere in der Kombination mit Gefriertrocknung vorteilhaft. Die vorliegende Erfindung umfaßt die Erkenntnis, daß die durch das Ausfällen der Fasem erzeugte Struktur, die durch den Einfriervorgang fixiert wird, bei der Entwässerung in Form der Gefriertrocknung im wesentlichen erhalten bleibt. Hierzu wird die auf die gewünschte Viskosität eingestellte und mit Fällungsmittei vermischte Suspension unter Berücksichtigung der angestrebten geometrischen Form bei Temperaturen unterhalb des Gefrierpunkts eingefroren. Je schneller der Einfriervorgang ist, desto feinporiger und gleichmäßiger wird der nach der Gefriertrocknung entstehende Schwamm. Das Einfrieren kann in üblichen Einfrierbädem oder auch in Kälteschränken unter Verwendung von verflüssigten Gasen, insbesondere flüssigem Stickstoff, als Kältemedium vorgenommen werden. Eine Zwischenlagerung der eingefrorenen Suspension bis zu mehreren Tagen oder Wochen ist prinzipiell möglich und hat keinen nachteiligen Einfluß auf die Qualität des Endprodukts.

Die Gefriertrocknung wird nach dem Stand der Technik durchgeführt, wie er beispielsweise von G.-W. Oetjen in **Gefriertrocknen, Wiley-VCH Verlag, 1997, 1.** Auflage, Weinheim zusammengefaßt ist. Es hat sich als vorteilhaft erwiesen, die Gefriertrocknung so durchzuführen, daß das gefrorene Material zu keinem Zeitpunkt an einer Stelle an- oder auftaut. Unter wirtschaftlichen Aspekten hat es sich als vorteilhaft erwiesen durch Zuführen von Energie z. B. über Strahlungswärme den Trocknungsvorgang zu beschleunigen. Zur Vermeidung von Verfärbungen ist es vorteilhaft, die Temperatur in den bereits getrockneten Teilen des Produkts höchstens so weit anzuheben, daß keine Produktschädigungen auftreten.

### Herstellung der Zubereitungen

Üblicherweise werden wäßrige Lösungen bzw. Suspensionen der Biopolymere mit einem Trockensubstanzgehalt von 0,1 bis 15, vorzugsweise 0,5 bis 10, insbesondere 1,0 bis 5,0 Gew.-% und besonders bevorzugt 1,5 bis 2,5 Gew.-% bei einem pH-Wert von 1,0 bis 12, vorzugsweise 4,0 bis 10 durch Zugabe von anorganischen oder organischen Säuren, vorzugsweise Salzsäure, Glykolsäure und/oder Milchsäure bzw. anorganischen oder organischen Basen, vorzugsweise Natriumhydrogencarbonat oder Ammoniak, hergestellt, wobei die Temperatur so gewählt werden sollte, daß sie die Quellung der Biopolymere unterstützt. Üblicherweise liegt diese im Bereich von 0 bis 100 °C und vorzugsweise 30 bis 65°C. Die auf diesem Wege hergestellten Suspensionen enthalten neben den gelösten Biopolymeren auch gequollene ungelöste Teilchen. Die durch die genannten Bedingungen eingestellte Viskosität der Suspension kann die späteren mechanischen Eigenschaften der Vliese beeinflussen.
Zur Verbesserung der Elastizität im getrockneten Zustand können den Suspensionen dann Polyole und weitere Hilfs- und Zusatzstoffe zugesetzt werden. Für die mechanischen Eigenschaften der Zubereitungen hat es sich außerdem als vorteilhaft erwiesen, den Suspensionen natürliche Fasern, wie beispielsweise Lignin, Polyose, Pektin und insbesondere Cellulose, oder aber Synthesefasern wie beispielsweise Polyester, Polyamide oder deren Gemische in einer Menge von 1 bis 50, vorzugsweise 5 bis 10 Gew.-% zuzusetzen. Besonders empfehlenswert ist es, die Fasern der Lösung bzw. der Suspension vor der Homogenisierung hinzu zugeben. Anschließend werden die Suspensionen homogenisiert. Nach der Herstellung der wässerigen Lösungen und/oder homogenisierten Suspensionen im gewünschten Viskositätsbereich werden diese in der Regel zur Vermeidung des Einschlusses von Gasbläschen, z.B. durch Vakuum oder Ultraschall entgast.

Die Zugabe und homogene Verteilung des Fällungsmittels kann so schnell (in der Regel 1 bis 10, bevorzugt 1 bis 4 Minuten) erfolgen, daß die Ausfällung und physikalische Vernetzung des Biopolymers überwiegend erst nach dem Befüllen der entsprechenden Einfrierform stattfindet. Für die Ausbildung der physikalischen Vernetzung hat es als besonders vorteilhaft erwiesen, das Produkt ohne weitere Durchmischung für 10 min bis 10 Stunden, insbesondere 30 min bis 6 h ruhen zu lassen. Das Fällungsmittel kann über ein Mischungselement mit statischen und/oder bewegten Einbauten zugemischt werden. Die resultierende Suspension kann in eine der beim Endprodukt gewünschten geometrischen Form entsprechende, geeignete Form gefüllt werden. Je nach gewünschter Form des Endproduktes kann es sich hierbei um Schalen, Rohre, Schläuche, Spritzen etc. handeln. Unterschiedliche Schichtdicken des Endproduktes können in Einfrierschalen durch die Füllhöhe der Suspension eingestellt werden. Für die Herstellung von Zubereitungen in Form von Vliesen, die z.B. als kosmetische Mittel oder Heilmittel oder Medizinprodukte eingesetzt werden, werden üblicherweise Schichtdicken 1 bis 100 mm, insbesondere von 15 bis 35 mm eingestellt.

Danach schließt sich üblicherweise eine Einfrierphase an, bevor die Zubereitung entwässert wird.

Die Zugabe weiterer Hilfs- und Zusatzstoffe kann sowohl vor der Zugabe des Fällungsmittels als auch zusammen mit dem Fällungsmittel erfolgen. Bevorzugt ist die Zugabe der weiteren Hilfs- und Zusatzstoffe vor der Zugabe des Fällungsmittels. Auch hier ist es von Vorteil die Lösungen bzw. Suspensionen mit den weiteren Hilfs- und Zusatzstoffen auf eine Viskosität im Bereich von 1000 bis 100 000, vorzugsweise im Bereich von 10 000 bis 40 000 mPas, insbesondere im Bereich von 15 000 bis 35 000 mPas einzustellen, bevor das Fällungsmittel zudosiert wird.

In einer weiteren Ausführungsform der Erfindung belädt man die vemetzerfreien Zubereitungen nach der Entwässerung mit Hilfs- und Zusatzstoffen. Hierbei werden beispielsweise kosmetische und pharmazeutische Wirkstoffe oder Aromastoffe mit speziellen Techniken auf die fertige, trockene Zubereitung nach der Gefriertrocknung aufgebracht. Dazu wird der Wirkstoff in einem geeigneten Lösungsmittel gelöst, auf den nach der Gefriertrocknung entstehenden Schwamm, der in dieser Ausführungsform als Trägermaterial fungiert, aufgebracht und das Lösungsmittel dann schonend entfernt. Geeignete Lösungsmittel sind beispielsweise überkritisches CO₂ oder unpolare oder polare organische Lösungsmittel, wie beispielsweise Hexan, Ethanol oder Isopropanol.
Die vorliegende Erfindung schließt die Erkenntnis mit ein, daß man in einer vemetzerfreien Zubereitung Hilfs- und Zusatzstoffe sowohl vor oder mit der Zugabe des Fällungsmittels als auch nach der Entwässerung zusetzen kann.

### Hitfs- und Zusatzstoffe

Als Hitfs- und Zusatzstoffe können Stoffe eingesetzt werden, die mit den vemetzerfreien Zubereitungen kompatibel sind und die physikalischen Eigenschaften der Zubereitungen positiv beeinflussen und/oder den Zubereitungen zusätzliche Funktionalitäten verleihen. Besonders bevorzugt als Hilfs- und Zusatzstoffe sind Stoffe, die ausgewählt sind aus der Gruppe bestehend aus Polyolen, Emulgatoren, Fasern, Farbstoffen, Parfümöle, Aromastoffen, kosmetischen Wirkstoffen, pharmazeutischen Wirkstoffen und Lebensmittelzusatzstoffen.

Die erfindungsgemäßen Zubereitungen können in untergeordneten Mengen als weitere Hilfs- und Zusatzstoffe Ölkörper, synthetische und natürliche Kohlenwasserstoffe, Wachse, kationische Polymere, Verdickungsmittel, Siliconverbindungen, biogene Wirkstoffe, Filmbildner, Konservierungsmittel, Solubilisatoren, Strukturbildner und Schutzlösungen (Cryoprotectant agents = CPA), UV-Lichtschutzfaktoren und dergleichen enthalten.

**Polyole,** die im Sinne der Erfindung als zusätzliche Bestandteile der vemetzerfreien Zubereitungen in Betracht kommen, besitzen vorzugsweise 2 bis 15 Kohlenstoffatome und mindestens zwei Hydroxylgruppen. Typische Beispiele sind :
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Üblicherweise werden die Polyole in Mengen von 0,1 bis 20, vorzugsweise 1 bis 10 Gew.-% - bezogen auf die Trockensubstanz der Biopolymere eingesetzt, wobei die Verwendung von Glycerin und Polyethylenglycolen bevorzugt ist.

Als **Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlensloffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglyce-rinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagetungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Üblicherweise werden die Emulgatoren in Mengen von 0,1 bis 20, vorzugsweise 1 bis 10 Gew.-%-bezogen auf die Trockensubstanz der Biopolymere eingesetzt.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fett-alkoholen und/oder Guerbetalkoholen mit aromatischen Carbonsäuren, insbesondere Benzoesäure, pflanzliche Öle, verzweigte primäre Alkohole, substituierte Cyclohexane, Guerbetcarbonate, Dialkylether, Siliconöle und/oder aliphatische bzw. naphthenische Kohlenwasserstoffe in Betracht.

Als **synthetische Kohlenwasserstoffe** können z.B. hydriertes Polyisobuten (synthetisches Squalan) , Polyisobuten, Polyethylen, Polypropylen eingesetzt werden. Als **natürliche Kohlenwasserstoffe** können Terpene, wie beispielsweise Squalen oder Squalan eingesetzt werden. Üblicherweise werden die Kohlenwasserstoffe in Mengen von 0,1 bis 20, vorzugsweise 1 bis 10 Gew.-% - bezogen auf die Trockensubstanz der Biopolymere eingesetzt.

Geeignete **Verdickungsmittel** sind beispielsweise Polysaccharide, insbesondere Xanthan-Gum, Guar-Guar, Agar-Agar, Alginate und Tylosen, Carboxymethylceliulose und Hydroxyethylcellulose, ferner höhermolekulare Polyethylenglycolmono- und -diester von Fettsäuren, Polyacrylate, Polyvinylalkohol und Polyvinylpyrrolidon.

Geeignete **kationische Polymere** sind beispielsweise kationische Cellulosederivate, kationische Stärke, Copolymere von Diallylammoniumsalzen und Acrylamiden, quatemierte Vinylpyrrolidon/Vinylimidazol-Polymere wie z.B. Luviquat® (BASF AG, Ludwigshafen/ FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), quatemierte Weizenpolypeptide, Polyethylenimin, kationische Siliconpolymere wie z.B. Amidomethicone oder Dow Coming, Dow Cor-ning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethytamino-1,3-propan, kationischer Guar-Gum wie z.B. Jaguar® CBS, Jaguar® C-17, Jaguar® C-16 der Celanese/US, quatemierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können.
Unter **biogenen Wirkstoffen** sind beispielsweise Bisabolol, Allantoin, Phytantriol, Panthenol, AHA-Säuren, Pflanzenextrakte, marine Extrakte, Vitamine und Vitaminkomplexe zu verstehen.

**Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.
Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 1 Gew.-%, bezogen auf die Trockensubstanz der Biopolymere eingesetzt.

Als **Fasern** können sowohl natürliche Fasern als auch Synthesefasem sowie Mischungen daraus eingesetzt werden. Als natürliche Fasem eignen sich beispielsweise Lignin, Polyose, Pektin und insbesondere Cellulose, als Synthesefasem eignen sich beispielsweise Polyester, Polyamide oder deren Gemische. Vorzugsweise werden die Fasem in einer Menge von 1 bis 50, vorzugsweise 5 bis 10 Gew.-% eingesetzt.

Unter **Wachsen** sind natürliche oder synthetische Stoffe zu verstehen, weiche bei 20°C knetbar, fest bis brüchig hart, grob bis feinkristallin, durchscheinend bis opak, jedoch nicht glasartig sind, oberhalb von 40°C ohne sich zu zersetzen schmelzen, schon wenig oberhalb des Schmelzpunktes niedrigviskos und nicht fadenziehend sind. Die im Sinne der Erfindung einzusetzenden Wachse unterscheiden sich beispielsweise von Harzen dadurch, daß sie in der Regel etwa zwischen und 50 und 90°C, in Ausnahmefällen auch bis zu 200°C, in den schmelzflüssigen, niedrigviskosen Zustand übergehen und praktisch frei von aschebildenden Verbindungen sind. Nach ihrer Herkunft teilt man die Wachse in die folgenden drei Gruppen ein: **Natürliche Wachse**, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; **chemisch modifizierte Wachse** (Hartwachse), wie z.B. Montanesterwachse, Sasolwachse, hydrierte Jojobawachse sowie synthetische Wachse, wie z.B. Polyalkylenwachse und Polyethylenglycolwachse. In diesem Zusammenhang ist der Einsatz von natürlichen Wachsen, speziell von pflanzlichen Wachsen bevorzugt.

**Aromastoffe** sind konzentrierte Zubereitungen von Geruchsstoffen oder Geschmacksstoffen, die dazu bestimmt sind, Lebensmitteln einen besonderen Geruch oder Geschmack zu verleihen. Beispiele sind Vanillin, Pfefferminzöl, Maillard-Produkte, Bananengeschmack und viele andere.
Wichtige Aromastoff-Träger sind die etherischen Öle, femer Abmischungen einzelner, im allgemeinen synthetisch hergestellter - man spricht hier von "naturidentischen" - Komponenten dieser Öle. Zur Zeit gibt es etwa 600 natürliche u. etwa 4200 naturidentische Aromastoffe für Nahrungsmittel, kosmetische Mitteln und pharmazeutische Produkte. Als Parfümöle seien beispielsweise genannt Gemische aus natürlichen und synthetischen Riechstoffen. Natürliche Riechstoffe sind Extrakte von Blüten, Stengeln und Blättem, Früchten, Fruchtschalen, Wurzeln, Hölzem, Kräutem und Gräsem, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylproplonat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

Als **Lebensmittelzusatzstoffe** werden Stoffe mit oder ohne Nährwert, die in der Regel weder selbst als Lebensmittel verzehrt noch als charakteristische Lebensmittelzutat verwendet werden und einem Lebensmittel aus technologischen Gründen bei der Herstellung, Verarbeitung, Zubereitung, Behandlung, Verpackung, Beförderung od. Lagerung zugesetzt werden, verstanden; wodurch sie selbst oder ihre Nebenprodukte zu Bestandteilen des Lebensmittels werden oder werden können. Einige Lebensmittelzusatzstoffe sind natürlicher Herkunft wie z.B. das Carotin aus Möhren, Chlorophyll aus grünen Pflanzen, Lecithin aus Eiern od. Sojabohnen. Andere dagegen sind rein synthetische Chemikalien wie z.B. die Azofarbstoffe Tartrazin u. Amaranth, die Antioxidantien BHA u. BHT u. die Süßstoffe Saccharin u. Cyclamat.

Unter **pharmazeutischen Wirkstoffen** sind Wirk- und Heilstoffe sowie deren Träger in den verschiedenen Arzneiformen umfaßt. Exemplarisch seien genannt Azelainsäure als Antiaknemittel oder PVP-Iod-Komplex zur Desinfektion.

Als **kosmetische Wirkstoffe** können alle Stoffe eingesetzt werden, die geeignet sind in kosmetischen Mittel eingesetzt zu werden.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
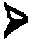 3-Benzylidencampher bzw. 3-Benzylidennorcampher und dessen Derivate, z.B. 3-(4-Methylbenzyliden)campher wie in der **EP 0693471 B1** beschrieben;
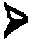 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
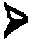 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
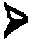 Ester der Salicylsäure, vorzugsweise Salicylsäure-2-ethylhexylester, Salicylsäure-4-isopropylbenzylester, Salicylsäurehomomenthylester,
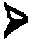 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
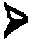 Ester der Benzalmalonsäure, vorzugsweise 4-Methoxybenzmalonsäuredi-2-ethylhexylester;
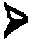 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
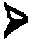 Propan-1,3-dione, wie z.B. 1-(4-tert.Butylphenyl)-3-(4'methoxyphenyl)propan-1,3-dion;
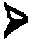 Ketotricyclo(5.2.1.0)decan-Derivate, wie in der **EP 0694521 B1** beschrieben.

Als wasserlösliche Substanzen kommen in Frage:
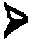 2-Phenylbenzimidazol-5-sulfonsäure und deren Alkali-, Erdalkali-, Ammonium-, Alkylammonium-, Alkanolammonium- und Glucammoniumsalze;
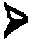 Sulfonsäurederivate von Benzophenonen, vorzugsweise 2-Hydroxy-4-methoxybenzophenon-5-sulfonsäure und ihre Salze;
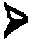 Sulfonsäurederivate des 3-Benrylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Buty)-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, **543** (1996) zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

**Cryoprotectant** agents sind beispielsweise Zuckerlösungen wie Sucrose, Maltose o.ä., Glycerin, PVP oder auch Pufferlösungen.

Der Gesamtanteil der Hilfs- und Zusatzstoffe kann 0,1 bis 50 vorzugsweise 0, 5 bis 10 Gew.-%-bezogen auf die Trockensubstanz der Biopolymere - betragen.

### Gewerbliche Anwendbarkeit

Die erfindungsgemäßen Zubereitungen zeichnen sich durch hohe Hautverträglichkeit und hohes Flüssigkeitsaufnahmevermögen aus. Ein weiterer Gegenstand der vorliegenden Erfindung betrifft deshalb die Verwendung der erfindungsgemäßen Zubereitungen als kosmetische Mittel, insbesondere als trockene Filme, Absorber, sowie kosmetische Masken und blutstillende Schwämme für kleine Schnittwunden z.B. verursacht durch Rasur.

Ein weiterer Gegenstand der Erfindung betrifft die Verwendung der erfindungsgemäßen Zubereitungen als Heilmittel und/oder Medizinprodukte, insbesondere als Wundtampons, Wundverbände, Brandwundverbände, wirkstoffabgebende Verbände, Vliese und als Drug Carrier für orale Applikationen. Dabei können die erfindungsgemäßen Zubereitungen mit verschiedenen topisch anwendbaren pharmazeutischen Formulierungen beladen werden. Für orale Applikationen können die erfindungsgemäßen Zubereitungen beispielsweise als Trägerstoff z.B. für Antibiotika, Schmerzmittel und andere dienen.

Ein weiterer Gegenstand der Erfindung betrifft die Verwendung der erfindungsgemäßen Zubereitungen als Lebensmittel. Unter Lebensmittel sind hierbei alle Stoffe zu verstehen, die dazu bestimmt sind, in unverändertem, zubereitetem oder verarbeitetem Zustand vom Menschen verzehrt zu werden. Hierunter sind insbesondere auch Nahrungsergänzungsmittel sowie diätetische Lebensmittel zu verstehen. Die erfindungsgemäßen Zubereitungen eignen sich darüber hinaus zur Verwendung als Lebensmittelzusatzstoffe.

## Patentansprüche

1. Vemetzerfreie dreidimensionale Strukturen, dadurch erhältlich, daß man wässrige. Lösungen und/oder homogenisierte Susoensionen von Biopolymeren mit einer Viskosität von 1 000 bis 100 000 mPas (gemessen nach Brookfield, Temperatur von 20°C) mit Fällungsmitteln versetzt und anschließend entwässert.

2. Verfahren zur Herstellung vemetzerfreier dreidimensionaler Strukturen, **dadurch gekennzeichnet, daß** man wässrige Lösungen und/oder homogenisierte Suspensionen von Biopolymeren mit einer Viskosität von 1 000 bis 100 000 mPas (gemessen nach Brookfield, Temperatur von 20°C) mit Fällungsmitteln versetzt und anschließend entwässert.

3. Verfahren nach Anspruch 2, **dadurch gekennzeichnet, daß** man 0,1 bis 15 Gew.-%ige wässrige Lösungen und/oder homogenisierte Suspensionen der Biopolymere einsetzt.

4. Verfahren nach mindestens einem der Ansprüche 2 bis 3, **dadurch gekennzeichnet, daß** die wässrigen Lösungen und/oder homogenisierten Suspensionen der Biopolymere einen pH-Wert von 1 bis 12 aufweisen.

5. Verfahren nach mindestens einem der Ansprüche 2 bis 4, **dadurch gekennzeichnet, daß** die Viskosität der wässrigen Lösungen und/oder homogenisierten Suspensionen der Biopolymere 10 000 bis 40 000 mPas beträgt.

6. Verfahren nach mindestens einem der vorgenannten Ansprüche 2 bis 5, **dadurch gekennzeichnet, daß** man Fällungsmittel einsetzt, die ausgewählt sind aus der Gruppe bestehend aus wässrigen Lösungen von Hydrogencarbonaten, Carbonaten, Hydrogenphosphaten und Hydroxiden der Alkaliund Erdalkalimetalle, Ammoniak und organischen Stickstoffbasen.

7. Verfahren nach Anspruch 6, **dadurch gekennzeichnet, daß** man als Fällungsmittel eine wässrige Natriumhydrogencarbonatlösung einsetzt.

8. Verfahren nach mindestens einem der vorgenannten Ansprüche 2 bis 7, **dadurch gekennzeichnet, daß** man Fällungsmittel einsetzt, die ausgewählt sind aus der Gruppe bestehend aus wässrigen Lösungen von Mineralsäuren und organischen Carbonsäuren.

9. Verfahren nach mindestens einem der vorgenannten Ansprüche 2 bis 8, **dadurch gekennzeichnet, daß** der pH-Wert der gefällten Biopolymere zwischen 1 und 14 liegt.

10. Verfahren nach mindestens einem der vorgenannten Ansprüche 2 bis 9, **dadurch gekennzeichnet, daß** man den wässrigen Lösungen und/oder homogenisierten Suspensionen vor oder mit der Zugabe des Fällungsmittels Hilfs- und Zusatzstoffe zumischt.

11. Verfahren nach mindestens einem der vorgenannten Ansprüche 2 bis 10, **dadurch gekennzeichnet, daß** man die vemetzerfreien Zubereitungen nach der Entwässerung mit Hilfs- und Zusatzstoffe belädt.

12. Verfahren nach den Ansprüchen 10 und/oder 11, **dadurch gekennzeichnet, daß** man als Hilfs- und Zusatzstoffe Stoffe einsetzt, die ausgewählt sind aus der Gruppe bestehend aus Polyolen, Emulgatoren, Fasem, Farbstoffen, Parfümöle, Aromastoffe, kosmetischen Wirkstoffen, pharmazeutischen Wirkstoffen und Lebensmittelzusatzstoffen.

13. Verfahren nach mindestens einem der vorgenannten Ansprüche 2 bis 12, **dadurch gekennzeichnet, daß** man mittels Gefriertrocknung entwässert.

14. Verwendung der vemetzerfreien Zubereitungen nach Anspruch 1 als kosmetische Mittel.

15. Verwendung der vemetzerfreien Zubereitungen nach Anspruch 1 als Heilmittel und/oder Medizinprodukte.

16. Verwendung der vemetzerfreien Zubereitungen nach Anspruch 1 als Lebensmittel.

17. Verwendung der vemetzerfreien Zubereitungen nach Anspruch 1 als Lebensmittelzusatzstoffe.

## Claims

1. Crosslinker-free three-dimensional structure obtainable by adding a precipitating agent to an aqueous solution and/or homogenized suspension of a biopolymer having a viscosity of from 1000 to 100 000 mPas (measured in accordance with Brookfield, temperature 20°C), and then dewatering the mixture.

2. Process for the preparation of crosslinker-free three-dimensional structures, **characterized in that** precipitating agents are added to aqueous solutions and/or homogenized suspensions of biopolymers having a viscosity of from 1000 to 100 000 mPas (measured in accordance with Brookfield, temperature 20°C), and then the mixtures are dewatered.

3. Process according to Claim 2, **characterized in that** 0.1 to 15% strength by weight aqueous solutions and/or homogenized suspensions of the biopolymers are used.

4. Process according to at least one of Claims 2 or 3, **characterized in that** the aqueous solutions and/or homogenized suspensions of the biopolymers have a pH of from 1 to 12.

5. Process according to at least one of Claims 2 to 4, **characterized in that** the viscosity of the aqueous solutions and/or homogenized suspensions of the biopolymers is 10 000 to 40 000 mPas.

6. Process according to at least one of the abovementioned Claims 2 to 5, **characterized in that** precipitating agents are used which are chosen from the group consisting of aqueous solutions of hydrogencarbonates, carbonates, hydrogenphosphates and the hydroxides of the alkali metals and alkaline earth metals, ammonia and organic nitrogen bases.

7. Process according to Claim 6, **characterized in that** the precipitating agent used is an aqueous sodium hydrogencarbonate solution.

8. Process according to at least one of the abovementioned Claims 2 to 7, **characterized in that** precipitating agents are used which are chosen from the group consisting of aqueous solutions of mineral acids and organic carboxylic acids.

9. Process according to at least one of the abovementioned Claims 2 to 8, **characterized in that** the pH of the precipitated biopolymers is between 1 and 14.

10. Process according to at least one of the abovementioned Claims 2 to 9, **characterized in that** auxiliaries and additives are mixed into the aqueous solutions and/or homogenized suspensions before or at the same time as the addition of the precipitating agent.

11. Process according to at least one of the abovementioned Claims 2 to 10, **characterized in that** the crosslinker-free preparations are charged with auxiliaries and additives after the dewatering.

12. Process according to Claims 10 and/or 11, **characterized in that** the auxiliaries and additives used are substances chosen from the group consisting of polyols, emulsifiers, fibres, dyes, perfume oils, aroma substances, cosmetic active ingredients, pharmaceutical active ingredients and food additives.

13. Process according to at least one of the abovementioned Claims 2 to 12, **characterized in that** dewatering is carried out by means of freeze-drying.

14. Use of the crosslinker-free preparations according to Claim 1 as cosmetic agents.

15. Use of the crosslinker-free preparations according to Claim 1 as medicaments and/or medicinal products.

16. Use of the crosslinker-free preparations according to Claim 1 as foods.

17. Use of the crosslinker-free preparations according to Claim 1 as food additives.

## Revendications

1. Structures tridimensionnelles sans agent réticulant, que l'on peut obtenir en mélangeant des solutions aqueuses et/ou des suspensions homogénéisées de biopolymères ayant une viscosité allant de 1000 à 100 000 mPas (mesurée selon Brookfield, à une température de 20°C) avec des agents de précipitation, puis en les déshydratant.

2. Procédé de préparation de structures tridimensionnelles sans agent réticulant,
**caractérisé en ce qu'**
on mélange des solutions aqueuses et/ou des suspensions homogénéisées de biopolymères ayant une viscosité de 1000 à 100 000 mPas (mesurée selon Brookfield, à une température de 20°C) avec des agents de précipitation, puis on les déshydrate.

3. Procédé selon la revendication 2,
**caractérisé en ce qu'**
on utilise de 0,1 à 15 % en poids de solutions aqueuses et/ou de suspensions homogénéisées des biopolymères.

4. Procédé selon au moins l'une des revendications 2 à 3,
**caractérisé en ce que**
les solutions aqueuses et/ou les suspensions homogénéisées des biopolymères présentent un pH de 1 à 12.

5. Procédé selon au moins l'une des revendications 2 à 4,
**caractérisé en ce que**
la viscosité des solutions aqueuses et/ou des suspensions homogénéisées des biopolymères est comprise entre 10 000 et 40 000 mPas.

6. Procédé selon au moins une des revendications 2 à 5 précédentes,
**caractérisé en ce qu'**
on utilise des agents de précipitation choisis dans le groupe constitué par des solutions aqueuses d'hydrogéno-carbonates, de carbonates, d'hydrogéno-phosphates et d'hydroxydes des métaux alcalins et alcalino-terreux, d'ammoniac et de bases azotées organiques.

7. Procédé selon la revendication 6,
**caractérisé en ce qu'**
on utilise comme agent de précipitation une solution aqueuse d'hydrogéno-carbonate de sodium.

8. Procédé selon au moins une des revendications 2 à 7 précédentes,
**caractérisé en ce qu'**
on utilise des agents de précipitation choisis dans le groupe constitué par des solutions aqueuses d'acides minéraux et d'acides carboxyliques organiques.

9. Procédé selon au moins une des revendications précédentes,
**caractérisé en ce que**
le pH des biopolymères précipités se situe entre 1 et 14.

10. Procédé selon au moins une des revendications 2 à 9 précédentes,
**caractérisé en ce qu'**
on ajoute des adjuvants et additifs aux solutions aqueuses et/ou aux suspensions homogénéisées, avant ou simultanément à l'addition de l'agent de précipitation.

11. Procédé selon au moins une des revendications 2 à 10 précédentes,
**caractérisé en ce qu'**
on charge les préparations sans agent réticulant avec des adjuvants et des additifs, après la déshydratation.

12. Procédé selon les revendications 10 et/ou 11,
**caractérisé en ce qu'**
on utilise comme adjuvants et additifs des matériaux choisis dans le groupe constitué par les polyols, les émulsifiants, les fibres, les colorants, les huiles parfumées, les substances aromatiques, les substances actives cosmétiques, les substances actives pharmaceutiques et les additifs alimentaires.

13. Procédé selon au moins une des revendications 2 à 12,
**caractérisé en ce qu'**
on effectue la déshydratation par lyophilisation.

14. Utilisation des préparations sans agent réticulant selon la revendication 1, comme agents cosmétiques.

15. Utilisation des préparations sans agent réticulant selon la revendication 1, comme médicaments et/ou produits médicinaux.

16. Utilisation des préparations sans agent réticulant selon la revendication 1, comme produits alimentaires.

17. Utilisation des préparations sans agent réticulant selon la revendication 1, comme additifs alimentaires.
